# EUROPEAN PATENT SPECIFICATION

(11) **EP 3 642 400 B1**
(45) Date of publication and mention of the grant of the patent: **07.12.2022**
(21) Application number: 17914781.4
(22) Date of filing: 11.12.2017
(51) Int. Cl.: C40B 50/06, C12Q 1/68

(54) **METHODS AND KITS OF CONSTRUCTING SEQUENCING LIBRARY FOR USE IN DETECTING CHROMOSOME COPY NUMBER VARIATION**
VERFAHREN UND KITS ZUR ERSTELLUNG EINER SEQUENZIERUNGSBIBLIOTHEK ZUR VERWENDUNG BEIM NACHWEIS VON VARIATIONEN DER CHROMOSOMENKOPIENZAHL
PROCÉDÉS ET KITS DE CONSTRUCTION D'UNE BIBLIOTHÈQUE DE SÉQUENÇAGE DESTINÉE À ÊTRE UTILISÉE DANS LA DÉTECTION D'UNE VARIATION DE NOMBRE DE COPIES DE CHROMOSOMES

(30) Priority: 21.06.2017 CN 201710476884
(43) Date of publication of application: 29.04.2020
(73) Proprietor: Berry Genomics Co., Ltd, Beijing 102206 (CN)
(72) Inventor: SHANG, Ling, Beijing 102206 (CN); LV, Meng, Beijing 102206 (CN); CHEN, Xiangbin, Beijing 102206 (CN); WANG, Tingting, Beijing 102206 (CN); ZHANG, Jianguang, Beijing 102206 (CN)
(74) Representative: Plasseraud IP
(86) International application number: PCT/CN2017/115378
(87) International publication number: WO 2018/233236

(56) References cited:
- WO-A1-2012/162884
- WO-A1-2013/104106
- WO-A1-2015/117040
- WO-A1-2015/196752
- WO-A2-2012/024658
- CN-A- 1 906 292
- CN-A- 102 108 406
- CN-A- 102 301 009
- CN-A- 107 217 308
- Roche: "KAPA HYPER PLUS Integrated Fragmentation and Library Preparation Solution Tunable and Reproducible Fragmentation", , 1 January 2015 (2015-01-01), XP055852189, Retrieved from the Internet: URL:https://www.n-genetics.com/products/11 04/1022/13602.pdf
- Louise Aigrain ET AL: "Quantitation of next generation sequencing library preparation protocol efficiencies using droplet digital PCR assays - a systematic comparison of DNA library preparation kits for Illumina sequencing", BMC Genomics, vol. 17, no. 1, 13 June 2016 (2016-06-13), XP055453168, DOI: 10.1186/s12864-016-2757-4

## Description

### Technical field

The present invention relates to methods and kits of constructing a library. More particularly, the present invention relates to methods and kits of constructing a sequencing library for use in detecting abnormality in chromosome copy number.

### Background

It has been reported that abnormality in chromosome number accounts for a great proportion of known abnormal chromosome-related diseases (Nagaoka et al., 2012). Abnormality in chromosome number can be characterized not only by changes in number of whole chromosomes (such as chromosome aneuploidy), but also by copy number variation (CNV) of certain fragments in certain chromosome (such as chromosome microdeletion microduplication syndrome). As diseases caused by chromosome abnormality usually has high incidence, result in serious harm and lack effective treatment, it is important to detect accurately whether the copy number of chromosome is abnormal by technical means.

Currently, conventional karyotyping in combination with fluorescence in situ hybridization (FISH) or chromosome microarray analysis (CMA, such as aCGH and SNP-array) are main techniques and standards used in clinical for detecting chromosome copy number variation (Nord et al., 2015). Karyotyping with high resolution, which was established in 1970s (Yunis, 1978; Trask, 2002), is still the golden standard in the detection of chromosome abnormality including chromosome aneuploidy, equilibrium or non-equilibrium structural rearrangement, deletion/duplication of large fragments, chimaera and the like. However, its resolution is relatively low (around 5Mb), and is unable to detect copy number variation of small fragments of a chromosome with clinical significance. On the other hand, the FISH technique, which started in late 1980s, combines cytogenetics, immunology and molecular biology, and has well application in clinical study for detecting target chromosome aneuploidy and structure abnormality. However, the design of specific probe in this technique is limited by the information obtained from the target region, thus only limited information about the chromosome can be obtained. Recently developed chromosome microarray analysis (CMA) is a molecular karyotyping technique for high throughput detection of genomic DNA copy number variation, wherein relatively matured comparative genomic hybridization (aCGH) and single nucleotide polymorphism (SNP) array both are CMA techniques (Manning and Hudgins, 2010). Although CMA has a high resolution compared with conventional karyotyping (Breman et al., 2012), in clinical application, the microarray chip must be customized according to detailed chromosomal abnormalities and related clinical information in the public database. Thus, it is insufficient for the detection of certain microdelection and/or microduplication that is rare or not included in the database. In addition, the current CMA technology is difficult and has a high cost, thereby limiting its broad application in less developed areas. Thus, in order to investigating and diagnosing chromosomal diseases more widely, there is a need for an economical and simple method of detecting chromosome copy number variation accurately while ensuring the detection throughput and resolution.

In recent years, the increasingly mature next generation sequencing (NGS) technology has been widely used in clinical studies due to its prominent advantages like high throughput, high accuracy, high sensitivity, high automation and low operation cost (Xuan et al., 2013). The most common principle employed in the detection of CNV by NGS is based on read-depth, i.e., determining the presence of CNV in certain fragments by calculating and comparing the depth of said fragments with the pre-calibrated theoretical value. This method applies to both single-end sequencing and paired-end sequencing (Yoon et al., 2009; Mason-Suares et al., 2016). During the construction of a NGS library, a standard PCR is often used to amplify random fragments, while the amplification bias brought by the PCR method will inevitably affect the accuracy of the obtained data (van Dijk et al., 2014; Head et al., 2014; Goodwin et al., 2016). Thus, it is critical to establish a simple PCR-free library construction method, thus to improve the detection accuracy of chromosome aneuploidy, in particular CNV.

Further background art is described in WO 2015/117040 A1, WO 2012/024658 A2, Roche, "KAPA HYPER PLUS Integrated Fragmentation and Library Preparation Solution Tunable and Reproducible Fragmentation", 2015, and Louise Aigrain et al., "Quantitation of next generation sequencing library preparation protocol efficiencies using droplet digital PCR assays - a systematic comparison of DNA library preparation kits for Illumina sequencing", 2016.

### Description of the Invention

The object of the present application is to provide a PCR-free method and kit of constructing a sequencing library for use in detecting variation in chromosome copy number, which overcomes the disadvantages of conventional methods for detecting the chromosome copy number variation in terms of resolution, coverage on the whole genome, throughput and cost, while addressing the problem of PCR amplification bias resulted in NGS library construction. Chromosome aneuploidy and chromosome microdeletion microduplication syndrome can be screened and diagnosed by using the method and kit of the present application to detect the chromosome copy number variation in a sample to be tested.

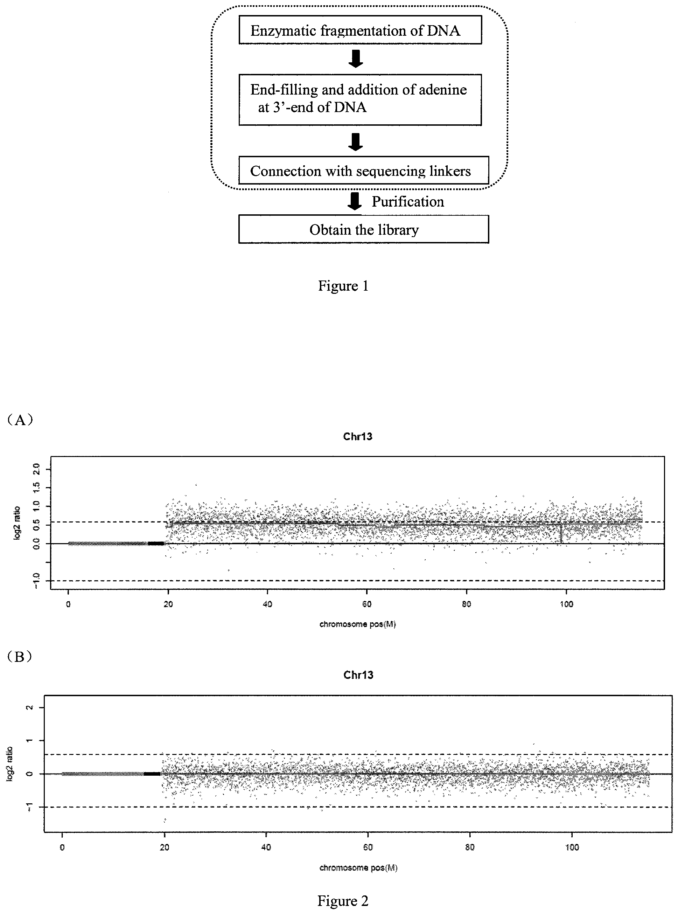

Thus, in a first aspect, the present application provides a method of constructing a sequencing library for use in detecting chromosome copy number variation, which mainly comprises the following steps:
(1) subjecting DNAs to be tested to random fragmentation by a double-strand DNA fragmentation enzyme;
(2) end-filling and adding poly-adenine tail at the 3' end of the fragmented DNAs;
(3) connecting the end-filled DNAs having a 3' end poly-adenine tail with linkers to obtain connected products;
(4) purifying the connected products to obtain the sequencing library;
wherein steps (1)-(3) are performed in a single reaction tube. Figure 1 shows the main steps of the method of the present application of constructing a sequencing library.

In one embodiment, the start amount of DNA to be tested preferably is 10-260ng. One skilled in the art knows that a suitable total amount of a library must not be less than 2fmol to meet the requirement of sequencing. Thus, to ensure the total amount of the constructed library to be at least equal to 2fmol, the inventors have found a preferable start amount of DNA to be tested of 10-260ng. If the amount is too low, the concentration of the obtained sequencing library will be not high enough to obtain effective sequencing data. On the other hand, if the amount is too high, the random fragmentation of DNAs to be tested will be inadequate. Thus, part of the DNAs to be tested still maintains its form of long fragment due to failure in fragmentation, and this part of DNAs cannot be effectively sequenced, which will have an impact on the concentration of the finally obtained library as well as the accuracy of its sequencing (see Example 2).

In one embodiment, the double-strand DNA fragmentation enzyme is an enzyme mixture of a nonspecific notch nuclease and a T7 endonuclease mutant. In particular, the nonspecific notch nuclease is a nuclease derived from Vibrio such as *Vibrio vulnificus* (Vvn), wherein said nuclease is wild-type or a mutant thereof. The T7 endonuclease mutant is a T7 endonuclease having a mutation or mutations for example in the bridging region of two catalytic domains as disclosed in U.S. publication No. 2007-0042379. In one embodiment, the double-strand DNA fragmentation enzyme comprising a nonspecific notch nuclease and a T7 endonuclease mutant is known to one skilled in the art, for example, as those disclosed in the patent publication CN102301009A.

In one embodiment, the unit ratio between the nonspecific notch nuclease and the T7 endonuclease mutant is less than 1:200, for example less than 1:100 or less than 1:10. The range thereof may be 1:2-1:200. Most preferably, the unit ratio between the nonspecific notch nuclease and the T7 endonuclease mutant is 1:200. One unit of T7 endonuclease mutant is defined as the amount of enzyme required to convert 90% of 2 µg of the linear-nicked 2.44 kb dsDNA into two fragments (1.37 kb and 1.07 kb) at 37°C for 1 hour. One unit of Vvn nuclease and mutants thereof is defined as the amount of enzyme required to release 1 A₂₆₀ unit of acid soluble oligonucleotides in 30 min at 37°C.

In one embodiment, the optimal incubation time for the DNA fragmentation reaction can be determined according to whether DNA has greater than 60% GC (high GC amount), 40-60% GC (standard GC amount) or less than 40% GC (low GC amount). For example, the incubation time typically may be in a range of 5-120min, for example 15-60min.

In one embodiment, the temperature of the DNA fragmentation reaction is 35-40 °C, most preferably 37°C.

In one embodiment, the random fragmentation of DNA to be detected is occurred in the presence of Buffer I, wherein said Buffer I mainly comprise 20mM trihydroxymethyl aminomethane-HCl (Tris-HCl), 15mM magnesium chloride (MgCl₂), 50mM sodium chloride (NaCl), 0.1mg/ml bovine serum albumin (BSA), and 0.15% Triton. The presence of Buffer I provide suitable reaction environment for the DNA fragmentation while maintaining the stability of the DNA fragmentation enzymes and improving the enzymatic activity thereof.

In one embodiment, there is a step of inactivating the double-strand DNA fragmentation enzymes between step (1) and step (2). The inactivation of the double-strand DNA fragmentation enzymes is carried out in a water bath at 60-70°C, such as 65°C for 10-13min, preferably in the presence of a buffer II, which comprises mainly 50mM NaCl, 10mM Tris-HCl, 10mM MgCl₂ and 1mM dithiothreitol(DTT). The presence of buffer II provides suitable ion concentrations for the inactivation of the enzyme, and also provides a suitable environment for the following reactions of end-filling and poly-adenine addition at 3'-end.

In one embodiment, any enzymes suitable for end-filling known by one skilled in the art can be used for end-filling of the present application. Examples of such enzymes include but not limited to T4 DNA polymerase, Klenow enzyme and the like. In one embodiment, any enzymes suitable for adding poly-adenine at 3'-end known by one skilled in the art can be used to add poly-adenine at 3'-end of the DNA. Examples of such enzymes include but not limited to the Taq enzyme, klenow ex-enzyme (an improved Klenow enzyme without 3'-5' exonuclease activity, New England Biolabs). In the method for constructing a library of the present invention, end-filling is carried out simultaneously with the poly-adenine addition, which simplifies the operation steps, saves the cost, while reducing possible contaminations among samples.

In one embodiment, one skilled in the art can determine the incubation time and temperature used in the end-filing and poly-adenine addition at 3'-end as needed by conventional technologies.

In one embodiment, any enzymes suitable for connecting a sequencing linker known by one skilled in the art can be used. Examples of such enzymes include but not limited to T4 DNA ligase, T7 DNA ligase or their mixtures. The sequencing linkers used in the present invention are double-strand sequencing linkers matched to the sequencing platform, and can be selected by one skilled in the art according to conventional techniques. Preferably, the connecting step is carried out in the presence of buffer III, which mainly comprises 50mM NaCl, 10mM Tris-HCl, 10mM MgCl₂ and 0.1% bovine serum albumin (BSA). The presence of buffer III provides a suitable environment for the connection reaction, and maintains the stability of the enzymes.

In one embodiment, a commercial kit of any reagents known by one skilled in the art can be used to purify the sequencing library obtained according to the construction method of the present application prior to sequencing. The obtained sequencing library is suitable for the second generation high-throughput sequencing platforms, such as HiSeq, MiSeq, MiniSeq, MySeq, NovaSeq sequencing platforms from Illumina, and PGM and Proton sequencing platforms from Thermo Fisher, and the like.

In a second aspect, the present invention provides a kit for constructing a sequencing library for use in detecting chromosome copy number variation, comprising: double-strand DNA fragmentation enzymes, buffer I, enzymes suitable for DNA end-filling, enzymes suitable for adding poly-adenine at 3' end, sequencing linkers, ligase and buffer III, wherein the DNA fragmentation, DNA end-filling and addition of poly-adenine at 3' end are carried out in a single reaction tube. The kit may further comprise buffer II. The composition of buffer I, II and III are as described above.

In one embodiment, the kit according to the present invention further comprises a DNA sample used as a negative control, which is a normal DNA sample does not comprise chromosome copy number variation.

The method for constructing a library according to the present invention basically is completed by enzymatic reactions. The properties of easy inactivation of enzymes and different reacting temperatures in different steps make it possible to sequentially and continuously carrying out the three steps of DNA random fragmentation, end-filling and poly-adenine addition at 3'-end in a single reaction tube without DNA purification between the steps. As the construction method according to the present invention can be practiced in a single reaction tube, it also avoids sample loss and reduces sample contamination resulted from DNA sample transfer. Further, as only a small number of reagents are involved, the construction method and kit of the present application allow a relatively simple operation procedure, and is easy for practice. Moreover, the construction method according to the present application does not comprise a PCR step, thus avoiding amplification bias brought by the PCR operation per se, thereby improving the accuracy of the detection. Finally, the construction method and kit according to the present application can detect a sample DNA as less as lOng accurately. That is, the initial amount of DNA sample required is low.

### Figures

Figure 1 is a diagram illustrating the method for constructing a library according to the present invention.
Figure 2 shows the sequencing results of a DNA sample to be tested (A) and a control normal sample (B) using libraries constructed according to the method of the present invention.
Figure 3 shows the sequencing results of a second DNA sample to be tested (A) and a control normal sample (B) using libraries constructed according to the method of the present invention.

### Examples

### Example 1: library construction according to the present invention and its use in DNA sequencing

This example detected the chromosome aneuploidy abnormality exemplified by Patau syndrome (T13) and chromosome microdeletion exemplified by chromosome 22q11.2 microdeletion syndrome, using the method and kit for constructing a a sequencing library for use in detecting chromosome copy number variation according to the present invention. Specifically, the Patau syndrome is a common trisomy syndrome, and is due to the presence of a third copy of chromosome 13. The chromosome 22q11.2 microdeletion syndrome is a clinical syndrome resulted from 22q11.21-q11.23 microdeletion in chromosome 22, and it is one of the most common microdeletion syndromes.

The sample DNAs are detected according to the following method.
1. determining concentration: the concentration of DNA samples to be tested was measured by Qubit florometer, and the initial amounts of DNA samples were adjusted preferably as 10-260 ng.
2. constructing a library: a sequencing library was constructed according to the method of the present application, which specifically comprises the following steps:
(1) preparing a reaction mixture on ice using DNA samples to be tested and a negative control (a normal DNA sample) as indicated in Table 1.

**Table 1:**

| Reagents | Amount |
|---|---|
| DNA sample | lOng |
| Double-stranded DNA fragmentation enzyme | 1µl |
| Buffer I | 1µl |
| EB buffer | add to 10µl |

After thorough mixing, the reaction mixture is incubated at 37°C for 10 min.
(2) The reaction mixture was transferred onto ice, added with 7µl buffer II, and incubated for 10min at 65 °C after mixing thoroughly, so as to inactive the double-stranded DNA fragmentation enzyme.
(3) The reaction mixture was transferred onto ice, added with 1.5µl T4 DNA polymerase and 1.5µl Taq enzyme, and then added to 50µl using the EB buffer. After thorough mixing, the reaction mixture was centrifuged for 5s at room temperature instantly, and bubbles were removed by slight flicks. Then, the reaction mixture was placed in a normal PCR machine (or a thermo metal bath) to carry out the following incubation steps: 20min at 37°C, followed by 20 min at 72°C, and finally 5 min at 4 °C.
(4) After incubation in the PCR machine (or a thermo metal bath), the reaction mixture was took out and placed on ice. Meanwhile, a pre-mixture is prepared according to Table 2.

**Table 2:**

| | Reagents | Amount |
|---|---|---|
| Buffer III | | 25µl |
| T4 DNA ligase | | 1µl |

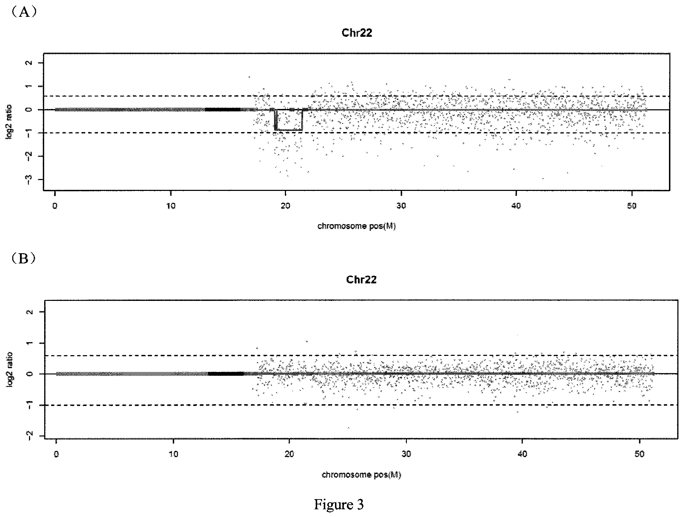

The pre-mixture was mixed thoroughly and centrifuged for 5s at room temperature instantly. Then, the pre-mixture was added to the incubated reaction mixture, and 2µl of sequencing linkers was added on the wall of the same reaction tube, followed by centrifugation for 5s at room temperature instantly. After rapid mixing, the mixture was centrifuged for 5s at room temperature instantly, and bubbles were removed by slight flicks. The mixture was centrifuged for 5s at room temperature instantly again. Then, the reaction mixture was placed in a normal PCR machine (or a thermo metal bath) to carry out the following incubation steps: 15min at 20°C, followed by 10 min at 65°C, and finally 5 min at 4 °C. The sequencing library was obtained after the incubation.
3. purifying the library and sequencing: the obtained sequencing library was purified using a purification kit from Hangzhou Berry Genomics Diagnostic Technology Ltd. (Lot No.: R0022), and the total amount is not less than 2fmol. Then, the purified sequencing library was loaded and sequenced on a NextSeq CN500 sequencing machine (National Medical Instrument Registration No.: 20153400460) according to the instructions of the manufactures.
4. analyzing the sequencing results: the sequencing results were aligned with the human genome reference sequences. The detection results of chromosome copy number in samples to be tested and in normal samples are presented in Figures 2 and 3.

In Figures 2 and 3, "ration" indicated in the Y-axis log₂(ratio) refers to the ratio between the copy number of the detected region and 2 (i.e., diploid), and the X-axis represents the whole chromosome region. The whole chromosome region was evenly divided into lots of bins, wherein the log₂(ratio) value of each bin corresponds to a single dot, and the distribution of all dots was used to obtain a trend line (the grey line). The black line in Figures 2 and 3 represents location of centromere in the chromosome, and separates the chromosome into a short arm and a long arm. As there are no reference sequences for the short arms of chromosomes 13 and 22 in the current human genome reference sequences that can be used for data analysis, the sequencing results related to the short arms of chromosomes 13 and 22 cannot be analyzed, resulting in the absence of corresponding log₂(ratio) values in these regions.

Specifically, Figure 2 shows the detection results of copy number in the chromosome 13 region of a DNA sample to be tested (A) and a normal sample (B). It is clear that for the DNA sample to be tested, the log₂(ratio) value of the long arm of chromosome 13 is around 0.585 (=log₂1.5), indicating that at least there are three copies for long arm part of the chromosome 13. That is, the DNA sample to be tested has Patau Sydrome (T13). Meanwhile, for the normal sample, the log₂(ratio) value of the long arm of chromosome 13 is 0(=log₂1), indicating that the chromosome 13 has only two copies. The DNA sample to be tested was detected by SNP-array, which confirmed the presence of three copies of chromosome 13 long arm. In other words, the detection results obtained using the library construction method and kit of the present invention are consistent with the clinical detection results.

The inventors also used the method and kit of the present invention to construct second libraries for a second DNA sample to be tested and a normal sample and then performed sequencing as indicated above. The results are shown in Figure 3. It is clear that for this DNA sample to be tested, the log₂(ratio) value of chromosome 22 in the 20M region is around -1(=log₂0.5), indicating that this region has only one copy. When aligned to the human genome reference sequences, this region with only one copy corresponds to the 22q11.21-q11.23 region. Thus, the DNA sample to be tested was confirmed to have a chromosome 22q11.2 microdeletion syndrome. Meanwhile, for the normal sample, the log₂(ratio) value of the long arm of chromosome 22 is 0(=log₂1), indicating that there is no deletion in 22q11.2 region of chromosome 22. The DNA sample to be tested was detected by SNP-array, which confirmed the presence of microdeletion in 22q11.2 region of chromosome 22. In other words, the detection results obtained using the library construction method and kit of the present invention are consistent with the clinical detection results.

This example shows that the library construction method and kit of the present invention can be used for constructing a sequencing library which allows the accurate detection of chromosome copy number variation by high throughput sequencing.

### Example 2: The effects of initial amount of DNA on the concentration of sequencing library

Samples with various initial amounts of DNA were used to prepare sequencing libraries according to the library construction and purification method of Example 1, and the total amount of obtained libraries were measured. The results are represented in Table 3.

**Table 3: The DNA initial amounts and the total amounts of finally obtained libraries**

| DNA initial amount (ng) | Total amount of library (fmol) | DNA initial amount (ng) | Total amount of library (fmol) |
|---|---|---|---|
| <5 | 0.142 | 150 | 2.752 |
| <5 | 0.2 | 200 | 2.348 |
| <5 | 0.21 | 200 | 1.782 |
| <5 | 0.108 | 220 | 2.462 |
| <5 | 0.129 | 220 | 1.758 |
| <5 | 0.169 | 240 | 2.078 |
| 5 | 1.053 | 240 | 1.935 |
| 5 | 1.592 | 260 | 2.149 |
| 10 | 2.776 | 260 | 2.228 |
| 10 | 2.401 | 280 | 1.405 |
| 50 | 2.97 | 280 | 1.585 |
| 50 | 2.988 | 290 | 1.982 |
| 100 | 2.318 | 290 | 1.812 |
| 100 | 3.11 | 300 | 1.04 |
| 150 | 2.477 | 300 | 0.568 |

Table 3 shows that when the DNA initial amount is between 10 and 260ng, the total amounts of the obtained libraries generally are greater than 2 fmol, which satisfies the requirements for sequencing. However, when the DNA initial amount is too low or too high, the total amounts of the obtained libraries are less than 2fmol, which is not optimal for sequencing. Thus, in the present invention, the DNA initial amount used for preparing a sequencing library preferably is 10-260n1.812g.

### References

Nagaoka SI, Hassold TJ, and Hunt PA. Human aneuploidy: mechanisms and new insights into an age-old problem. Nat. Rev. Genet. 2012, 13: 493e504
Nord A, Salipante SJ, Pritchard C. Chapter 11 - Copy Number Variant Detection Using Next-Generation Sequencing. Clinical Genomics, 2015 , 8 :165-187
Yunis J. High-resolution chromosome analysis in clinical medicine. Prog. Clin. Pathol., 1978, 7: 267-288
Trask BJ. Human cytogenetics: 46 chromosomes, 46 years and counting. Nat. Rev. Genet. 2002, 3: 769e778
Trask BJ. Fluoresence in situ hybridization: application in cytogenetics and gene mapping. Trends Gnent. 1991, 7:149-154.
Manning M, and Hudgins L. Array-based technology and recommendations for utilization in medical genetics practice for detection of chromosomal abnormalities. Genet. Med. 2010, 12(11):742-745.
Breman A, Pursley AN, Hixson P, Bi W, Ward P, Bacino CA, Shaw C, Lupski JR, Beaudet A, Patel A, Cheung SW, Van den Veyver I: Prenatal chromosomal microarray analysis in a diagnostic laboratory : experience with >1000 cases and review of the literature. Prenat. Diagn. 2012, 32: 351e361.
Xuan J, Yu Y, Qing T, Guo L, Shi L. Next-generation sequencing in the clinic: promises and challenges. Cancer Lett. 2013, 340(2):284-295.
Yoon S, Xuan Z, Makarov V, Ye K, Sebat J. Sensitive and accurate detection of copy number variants using read depth of coverage. Genome Res. 2009, 19(9):1586-1592.
Mason-Suares H, Landry L, Lebo MS. Detecting Copy Number Variation via Next Generation Technology. Curr. Genet. Med. Report. 2016, 4 (3): 1-12.
van Dijk EL, Jaszczyszyn Y, Thermes C. Library preparation methods for next-generation sequencing: tone down the bias. Exp. Cell Res. 2014, 322(1):12-20.
Head SR, Komori HK, LaMere SA, Whisenant T, Van Nieuwerburgh F, Salomon DR, Ordoukhanian P1.Library construction for next-generation sequencing: overviews and challenges. Biotechniques. 2014, 56(2):61-64.
Goodwin S, McPherson JD, McCombie WR. Coming of age: ten years of next-generation sequencing technologies. Nat. Rev. Genet. 2016, 17(6):333-351.

## Claims

1. A method of rapidly constructing a high throughput sequencing library for detecting chromosome copy number variation, **characterized in that**, the method comprises the following steps:
(1) subjecting DNAs to be tested to random fragmentation by a double-strand DNA fragmentation enzyme;
(2) end-filling and adding poly-adenine at the 3' end of the fragmented DNAs;
(3) connecting the end-filled DNAs having a 3' end poly-adenine with sequencing linkers to obtain connected products;
(4) purifying the connected products to obtain the sequencing library;
wherein steps (1)-(3) are performed in a single reaction tube;
wherein the DNAs are as less as lOng; and
wherein the method does not comprise a step of PCR amplification.

2. The method according to claim 1, **characterized in that**, there is no DNA purification steps among steps (1)-(3).

3. The method according to claim 1, **characterized in that**, there is a step of inactivating the double-strand DNA fragmentation enzymes between the step (1) and step (2).

4. The method according to claim 1, **characterized in that**, the double-strand DNA fragmentation enzyme is an enzyme mixture of a nonspecific notch nuclease and a T7 endonuclease mutant.

5. The method according to claim 3, **characterized in that**, the nonspecific notch nuclease is a Vvn nuclease, and the T7 endonuclease mutant is a T7 endonuclease having a mutation or mutations in the bridging region of two catalytic domains.

6. The method according to claim 1, **characterized in that**, step (2) is carried out by T4 DNA polymerase and Taq enzyme.

7. The method according to claim 1, **characterized in that**, the sequencing linkers are double-strand sequencing linkers matched to sequencing platforms.

8. The method according to claim 1, **characterized in that**, step (3) is carried out by T4 DNA ligase.

9. The method according to claim 1, **characterized in that**, the sequencing library is suitable for the second generation high throughput sequencing platforms.

10. A kit for constructing a sequencing library for use in PCR-free detection of chromosome copy number variation, **characterized in that**, the kit comprises: double-strand DNA fragmentation enzymes and buffer I for DNA fragmentation, enzymes for DNA end-filling, enzymes for adding poly-adenine at 3' end, sequencing linkers, ligase and buffer III for ligation, wherein the DNA fragmentation, DNA end-filling and addition of poly-adenine at 3' end are carried out in a single reaction tube, and wherein the DNAs are as less as lOng, **characterized in that**, the double-strand DNA fragmentation enzyme is an enzyme mixture of a nonspecific notch nuclease and a T7 endonuclease mutant.

11. The kit according to claim 10, **characterized in that**, the nonspecific notch nuclease is a Vvn nuclease, and the T7 endonuclease mutant is a T7 endonuclease having a mutation or mutations in the bridging region of two catalytic domains.

12. The kit according to claim 10, **characterized in that**, the enzyme for DNA end-filling is T4 DNA polymerase, and the enzyme for adding poly-adenine at 3' end is Taq enzyme.

13. The kit according to claim 10, **characterized in that**, it further comprises buffer II for inactivating the double-strand DNA fragmentation enzymes.

## Patentansprüche

1. Verfahren zum schnellen Aufbau einer Hochdurchsatz-Sequenzierungsbibliothek zum Nachweisen einer Chromosomenkopienzahlvariation, **dadurch gekennzeichnet, dass** das Verfahren die folgenden Schritte umfasst:
(1) Unterziehen der zu testenden DNAs einer zufälligen Fragmentierung durch ein Doppelstrang-DNA-Fragmentierungsenzym;
(2) Endauffüllen und Hinzufügen von Polyadenin am 3'-Ende der fragmentierten DNAs;
(3) Verbinden der endgefüllten DNAs, die ein Polyadenin am 3'-Ende aufweisen, mit Sequenzierungslinkern, um verbundene Produkte zu erhalten;
(4) Reinigen der verbundenen Produkte, um die Sequenzierungsbibliothek zu erhalten;
wobei Schritte (1) - (3) in einem einzigen Reaktionsgefäß durchgeführt werden;
wobei die DNAs weniger als 10 ng sind; und
wobei das Verfahren keinen Schritt von PCR-Amplifikation umfasst.

2. Verfahren nach Anspruch 1, **dadurch gekennzeichnet, dass** es zwischen den Schritten (1) - (3) keine DNA-Reinigungsschritte gibt.

3. Verfahren nach Anspruch 1, **dadurch gekennzeichnet, dass** es zwischen Schritt (1) und Schritt (2) einen Schritt von Inaktivierung der Doppelstrang-DNA-Fragmentierungsenzyme gibt.

4. Verfahren nach Anspruch 1, **dadurch gekennzeichnet, dass** das Doppelstrang-DNA-Fragmentierungsenzym eine Enzymmischung aus einer unspezifischen Notch-Nuklease und einer T7-Endonuklease-Mutante ist.

5. Verfahren nach Anspruch 3, **dadurch gekennzeichnet, dass** die unspezifische Notch-Nuklease eine Vvn-Nuklease ist, und die T7-Endonuklease-Mutante eine T7-Endonuklease mit einer Mutation oder Mutationen in der Brückenregion von zwei katalytischen Domänen ist.

6. Verfahren nach Anspruch 1, **dadurch gekennzeichnet, dass** Schritt (2) durch T4-DNA-Polymerase und Taq-Enzym durchgeführt wird.

7. Verfahren nach Anspruch 1, **dadurch gekennzeichnet, dass** die Sequenzierungslinker Doppelstrang-Sequenzierungslinker sind, die auf Sequenzierungsplattformen abgestimmt sind.

8. Verfahren nach Anspruch 1, **dadurch gekennzeichnet, dass** Schritt (3) durch T4-DNA-Ligase durchgeführt wird.

9. Verfahren nach Anspruch 1, **dadurch gekennzeichnet, dass** die Sequenzierungsbibliothek für die Hochdurchsatz-Sequenzierungsplattformen der zweiten Generation geeignet ist.

10. Kit zum Aufbau einer Sequenzierungsbibliothek zur Verwendung beim PCRfreien Nachweis von Chromosomenkopienzahlvariationen, **dadurch gekennzeichnet, dass** der Kit Doppelstrang-DNA-Fragmentierungsenzyme und Puffer I für DNA-Fragmentierung, Enzyme für DNA-Endauffüllung, Enzyme zum Hinzufügen von Polyadenin am 3'-Ende, Sequenzierungslinker, Ligase und Puffer III für Ligation umfasst, wobei die DNA-Fragmentierung, DNA-Endauffüllung und Hinzufügen von Polyadenin am 3'-Ende in einem einzigen Reaktionsröhrchen durchgeführt werden, und wobei die DNAs weniger als 10 ng sind, **dadurch gekennzeichnet, dass** das Doppelstrang-DNA-Fragmentierungsenzym eine Enzymmischung aus einer unspezifischen Notch-Nuklease und einer T7-Endonuklease-Mutante ist.

11. Kit nach Anspruch 10, **dadurch gekennzeichnet, dass** die unspezifische Notch-Nuklease eine Vvn-Nuklease ist und die T7-Endonuklease-Mutante eine T7-Endonuklease mit einer Mutation oder Mutationen in der Brückenregion von zwei katalytischen Domänen ist.

12. Kit nach Anspruch 10, **dadurch gekennzeichnet, dass** das Enzym zum DNA-Endauffüllen T4-DNA-Polymerase ist und das Enzym zum Hinzufügen von Polyadenin am 3'-Ende Taq-Enzym ist.

13. Kit nach Anspruch 10, **dadurch gekennzeichnet, dass** er ferner Puffer II zur Inaktivierung der Doppelstrang-DNA-Fragmentierungsenzyme umfasst.

## Revendications

1. Procédé de construction rapide d'une bibliothèque de séquençage à haut débit pour détection de la variation du nombre de copies chromosomiques, **caractérisé en ce que** le procédé comprend les étapes suivantes :
(1) soumission des ADN à tester à une fragmentation aléatoire par une enzyme de fragmentation d'ADN double brin ;
(2) remplissage aux extrémités et ajout de poly-adénine à l'extrémité 3' des ADN fragmentés ;
(3) connexion des ADN remplis aux extrémités ayant une poly-adénine à l'extrémité 3' avec des lieurs de séquençage pour obtenir des produits connectés ;
(4) purification des produits connectés pour obtenir la bibliothèque de séquençage ;
les étapes (1) à (3) étant réalisées dans un seul tube de réaction ;
les ADN étant en quantité aussi faible que 10ng ; et
le procédé ne comprenant pas d'étape d'amplification par PCR.

2. Procédé selon la revendication 1, **caractérisé en ce qu'**il n'y a pas d'étape de purification d'ADN parmi les étapes (1) à (3).

3. Procédé selon la revendication 1, **caractérisé en ce qu'**il y a une étape d'inactivation des enzymes de fragmentation d'ADN double brin entre l'étape (1) et l'étape (2).

4. Procédé selon la revendication 1, **caractérisé en ce que** l'enzyme de fragmentation d'ADN double brin est un mélange enzymatique d'une nucléase notch non spécifique et d'un mutant de l'endonucléase T7.

5. Procédé selon la revendication 3, **caractérisé en ce que** la nucléase notch non spécifique est une nucléase Vvn, et que le mutant de l'endonucléase T7 est une endonucléase T7 ayant une mutation ou des mutations dans la région de pontage de deux domaines catalytiques.

6. Procédé selon la revendication 1, **caractérisé en ce que** l'étape (2) est effectuée par de l'ADN polymérase T4 et de l'enzyme Taq.

7. Procédé selon la revendication 1, **caractérisé en ce que** les lieurs de séquençage sont des lieurs de séquençage double brin mis en correspondance avec des plateformes de séquençage.

8. Procédé selon la revendication 1, **caractérisé en ce que** l'étape (3) est effectuée par de l'ADN ligase T4.

9. Procédé selon la revendication 1, **caractérisé en ce que** la bibliothèque de séquençage est adaptée aux plateformes de séquençage à haut débit de seconde génération.

10. Kit de construction d'une bibliothèque de séquençage à utiliser dans une détection exempte de PCR d'une variation du nombre de copies chromosomiques, **caractérisé en ce que** le kit comprend : des enzymes de fragmentation d'ADN double brin et tampon 1 pour une fragmentation d'ADN, des enzymes pour le remplissage aux extrémités de l'ADN, des enzymes pour l'ajout de poly-adénine à l'extrémité 3', des lieurs de séquençage, de la ligase et du tampon III pour une ligature ; la fragmentation d'ADN, le remplissage aux extrémités de l'ADN et l'ajout de poly-adénine à l'extrémité 3' étant effectués dans un seul tube de réaction, et les ADN étant en quantité aussi faible que lOng, **caractérisé en ce que** l'enzyme de fragmentation d'ADN double brin est un mélange enzymatique d'une nucléase notch non spécifique et d'un mutant de l'endonucléase T7.

11. Kit selon la revendication 10, **caractérisé en ce que** la nucléase notch non spécifique est une nucléase Vvn et le mutant de l'endonucléase T7 est une endonucléase T7 ayant une mutation ou des mutations dans la région de pontage de deux domaines catalytiques.

12. Kit selon la revendication 10, **caractérisé en ce que** l'enzyme pour le remplissage aux extrémités de l'ADN est l'ADN polymérase T4, et l'enzyme pour l'ajouter de poly-adénine à l'extrémité 3' est l'enzyme Taq.

13. Kit selon la revendication 10, **caractérisé en ce qu'**il comprend en outre du tampon II pour inactiver les enzymes de fragmentation d'ADN double brin.
